# EUROPEAN PATENT SPECIFICATION

(11) **EP 1 042 327 B1**
(45) Date of publication and mention of the grant of the patent: **17.09.2003**
(21) Application number: 98959652.3
(22) Date of filing: 01.12.1998
(51) Int. Cl.: C07D 493/04, C07D 417/06, C07D 277/24, C07D 493/08, A61K 31/425

(54) **A PROCESS FOR THE REDUCTION OF OXIRANYL EPOTHILONES TO OLEFINIC EPOTHILONES**
VERFAHREN ZUR REDUKTION VON OXIRANYL-EPOTHILONEN ZU OLEFINISCHEN EPOTHILONEN
PROCEDE DE REDUCTION D'EPOTHILONES D'OXIRANYLE EN EPOTHILONES OLEFINIQUES

(30) Priority: 04.12.1997 US 67549 P; 21.04.1998 US 82563 P
(43) Date of publication of application: 11.10.2000
(73) Proprietor: BRISTOL-MYERS SQUIBB COMPANY, Princeton, NJ 08543-4000 (US)
(72) Inventor: KIM, Soong-Hoon, Lawrenceville, NJ 08648 (US); JOHNSON, James, A., Lawrenceville, NJ 08648 (US)
(74) Representative: VOSSIUS & PARTNER
(86) International application number: US9825464
(87) International publication number: WO99028324

(56) References cited:
- WO-A1-93/10121
- WO-A1-97/19086
- MENG D ET AL: "Total syntheses of epothilones A and B" JOURNAL OF THE AMERICAN CHEMICAL SOCIETY, vol. 119, no. 42, 22 October 1997 (1997-10-22), pages 10073-10092, XP002122507
- SCHOBERT et al., "Reduction and Isomerization of Oxiranes and alpha-Diazoketones by Various Early Transition Metallocenes", SYNLETT, August 1990, No. 8, pages 465-466, XP002916072
- SHARPLESS et al., "Lower Valent Tungsten Halides. A New Class of Reagents for Deoxygenation of Organic Molecules", JOURNAL OF THE AMERICAN CHEMICAL SOCIETY, 06 September 1972, Vol. 94, No. 18, pages 6538-6540, XP002916073
- NICOLAOU et al., "Designed Epothilones: Combinatorial Synthesis, Tubulin Assembly Properties and Cytotoxic Action Against Taxol-Resistant Tumor Cells", ANGEW. CHEM. INT. ED. ENGL., 1997, Vol. 36, No. 19, pages 2097-2102, XP002916074
- SU et al., "Structure - Activity Relationships of the Epothilones and the First In Vivo Comparison with Paclitaxel", ANGEW. CHEM. INT. ED. ENGL., 1997, Vol. 36, No. 19, pages 2093-2096, XP002916075

## Description

The present invention is directed to a process for preparing compounds of formulas II and IV. 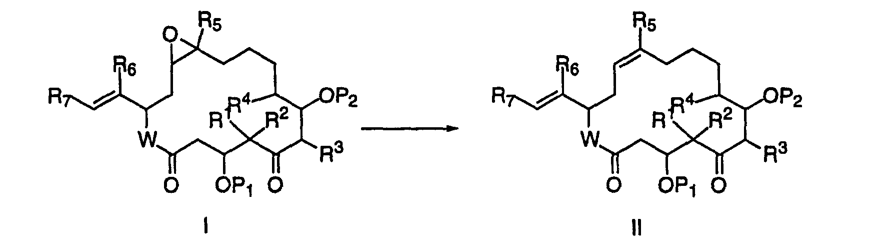 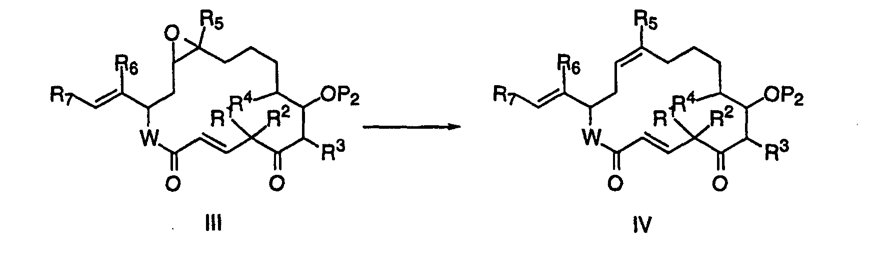 The compounds of formulas I - IV are useful in the treatment of a variety of cancers and other abnormal proliferative diseases. Compounds of formula I are disclosed in Hofle et al., Angew. Chem. Int. Ed. Engl., 1996, 35, 1567; WO93/10121 published May 27, 1993 and WO97/19086 published May 29, 1997 and also Nicolaou et al., Angew Chem. Int. Ed. Engl., 1997, 36, 2097 and Danishefsky et al., Angew Chem. Int. Ed. Engl., 1997, 36, 2093. The chemical construction of the C12-C13 double bond in epothilones by metathesis is disclosed in Meng et al., J. Am. Chem. Soc. **1997,** *119*, 10073. As used in the formulas I and II, and throughout the specification, the symbols have the following meanings:
W is O, NR₈;
R₁, R₂, R₃, R₄, R₅, R₆, are selected from the group H, alkyl, substituted alkyl, or aryl and when R₁ and R₂ are alkyl can be joined to form a cycloalkyl;
R₇ is selected from the group consisting of H, alkyl, substituted alkyl, aryl, cycloalkyl, or heterocyclo;
R₈ is H, alkyl, or substituted alkyl, OH, O-alkyl, O-substituted alkyl;
P₁ and P₂ are selected from the group, H, alkyl, substituted alkyl, alkanoyl, substituted alkanoyl, aroyl, substituted aroyl, trialkylsilyl, aryl dialkylsilyl, diaryl alkylsilyl, triarylsilyl.

### Detailed Description of the Invention

Listed below are definitions of various terms used to describe this invention. These definitions apply to the terms as they are used throughout this specification, unless otherwise limited in specific instances, either individually or as part of a larger group.

The term "alkyl" refers to straight or branched chain unsubstituted hydrocarbon groups of 1 to 20 carbon atoms, preferably 1 to 7 carbon atoms.

The term "substituted alkyl" refers to an alkyl group substituted by, for example, one to four substituents, such as, halo, trifluoromethyl, trifluoromethoxy, hydroxy, alkoxy, cycloalkyoxy, heterocylooxy, oxo, alkanoyl, aryloxy, alkanoyloxy, amino, alkylamino, arylamino, aralkylamino, cycloalkylamino, heterocycloamino, disubstituted amines in which the 2 amino substituents are selected from alkyl, aryl or aralkyl, alkanoylamino, aroylamino, aralkanoylamino, substituted alkanoylamino, substituted arylamino, substituted aralkanoylamino, thiol, alkylthio, arylthio, aralkylthio, cycloalkylthio, heterocyclothio, alkylthiono, arylthiono, aralkylthiono, alkylsulfonyl, arylsulfonyl, aralkylsulfonyl, sulfonamido (e.g. SO₂NH₂), substituted sulfonamido, nitro, cyano, carboxy, carbamyl (e.g. CONH₂), substituted carbamyl (e.g. CONH alkyl, CONH aryl, CONH aralkyl or cases where there are two substituents on the nitrogen selected from alkyl, aryl or aralkyl), alkoxycarbonyl, aryl, substituted aryl, guanidino and heterocyclos, such as, indolyl, imidazolyl, furyl, thienyl, thiazolyl, pyrrolidyl, pyridyl, pyrimidyl and the like. Where noted above where the substituent is further substituted it will be with halogen, alkyl, alkoxy, aryl or aralkyl.

The term "halogen" or "halo" refers to fluorine, chlorine, bromine and iodine.

The term "aryl" refers to monocyclic or bicyclic aromatic hydrocarbon groups having 6 to 12 carbon atoms in the ring portion, such as phenyl, naphthyl, biphenyl and diphenyl groups, each of which may be substituted.

The term "aralkyl" refers to an aryl group bonded directly through an alkyl group, such as benzyl.

The term "substituted aryl" refers to an aryl group substituted by, for example, one to four substituents such as alkyl; substituted alkyl, halo, trifluoromethoxy, trifluoromethyl, hydroxy, alkoxy, cycloalkyloxy, heterocyclooxy, alkanoyl, alkanoyloxy, amino, alkylamino, aralkylamino, cycloalkylamino, heterocycloamino, dialkylamino, alkanoylamino, thiol, alkylthio, cycloalkylthio, heterocyclothio, ureido, nitro, cyano, carboxy, carboxyalkyl, carbamyl, alkoxycarbonyl, alkylthiono, arylthiono, alkysulfonyl, sulfonamido, aryloxy and the like. The substituent may be further substituted by halo, hydroxy, alkyl, alkoxy, aryl, substituted aryl, substituted alkyl or aralkyl.

The term "cycloalkyl" refers to a optionally substituted, saturated cyclic hydrocarbon ring systems, preferably containing 1 to 3 rings and 3 to 7 carbons per ring which may be further fused with an unsaturated C₃-C₇ carbocyclic ring. Exemplary groups include cyclopropyl, cyclobutyl, cyclopentyl, cyclohexyl, cycloheptyl, cyclooctyl, cyclodecyl, cyclododecyl, and adamantyl. Exemplary substituents include one or more alkyl groups as described above, or one or more groups described above as alkyl substituents.

The terms "heterocycle", "heterocyclic" and "heterocyclo" refer to an optionally substituted, fully saturated or unsaturated, aromatic or nonaromatic cyclic group, for example, which is a 4 to 7 membered monocyclic, 7 to 11 membered bicyclic, or 10 to 15 membered tricyclic ring system, which has at least one heteroatom in at least one carbon atom-containing ring. Each ring of the heterocyclic group containing a heteroatom may have 1, 2 or 3 heteroatoms selected from nitrogen atoms, oxygen atoms and sulfur atoms, where the nitrogen and sulfur heteroatoms may also optionally be oxidized and the nitrogen heteroatoms may also optionally be quaternized. The heterocyclic group may be attached at any heteroatom or carbon atom.

Exemplary monocyclic heterocyclic groups include pyrrolidinyl, pyrrolyl, indolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolidinyl, 2-oxazepinyl, azepinyl, 4-piperidonyl, pyridyl, N-oxo-pyridyl, pyrazinyl, pyrimidinyl, pyridazinyl, tetrahydropyranyl, tetrahydrothiopyranyl, tetrahydrothiopyranyl sulfone, morpholinyl, thiomorpholinyl, thiomorpholinyl sulfoxide, thiomorpholinyl sulfone, 1,3-dioxolane and tetrahydro-1, 1-dioxothienyl, dioxanyl, isothiazolidinyl, thietanyl, thiiranyl, triazinyl, and triazolyl, and the like.

Exemplary bicyclic heterocyclic groups include benzothiazolyl, benzoxazolyl, benzothienyl, quinuclidinyl, quinolinyl, quinolinyl-N-oxide, tetrahydroisoquinolinyl, isoquinolinyl, benzimidazolyl, benzopyranyl, indolizinyl, benzofuryl, chromonyl, coumarinyl, cinnolinyl, quinoxalinyl, indazolyl, pyrrolopyridyl, furopyridinyl (such as furo[2,3-c]pyridinyl, furo[3,1-b]pyridinyl] or furo[2,3-b]pyridinyl), dihydroisoindolyl, dihydroquinazolinyl (such as 3,4-dihydro-4-oxoquinazolinyl), benzisothiazolyl, benzisoxazolyl, benzodiazinyl, benzofurazanyl, benzothiopyranyl, benzotriazolyl, benzpyrazolyl, dihydrobenzofuryl, dihydrobenzothienyl, dihydrobenzothiopyranyl, dihydrobenzothiopyranyl sulfone, dihydrobenzopyranyl, indolinyl, isochromanyl, isoindolinyl, naphthyridinyl, phthalazinyl, piperonyl, purinyl, pyridopyridyl, quinazolinyl, tetrahydroquinolinyl, thienofuryl, thienopyridyl, thienothienyl, and the like.

Exemplary substituents include one or more alkyl groups as described above or one or more groups described above as alkyl substituents. Also included are smaller heterocyclos, such as, epoxides and aziridines.
The term "alkanoyl" refers to -C(O)-alkyl.
The term "substituted alkanoyl" refers to -C(O)-substituted alkyl.
The term "aroyl" refers to -C(O)-aryl.
The term "substituted aroyl" refers to -C(O)-substituted aryl.
The term "trialkylsilyl" refers to -Si(alkyl)₃.
The term "aryl dialkylsilyl" refers to -Si(alkyl)₂(aryl).
The term "diaryl alkylsilyl" refers to -Si(aryl)₂(alkyl).
The term "heteroatoms" shall include oxygen, sulfur and nitrogen.

### Use and Utility

The compounds of formula I - IV are microtubule-stabilizing agents. They are thus useful in the treatment of a variety of cancers, including (but not limited to) the following;
- carcinoma, including that of the bladder, breast, colon, kidney, liver, lung, ovary, pancreas, stomach, cervix, thyroid and skin; including squamous cell carcinoma;
- hematopoietic tumors of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma and Burketts lymphoma;
- hematopoietic tumors of myeloid lineage, including acute and chronic myelogenous leukemias and promyelocytic leukemia;
- tumors of mesenchymal origin, including fibrosarcoma and rhabdomyoscarcoma;
- other tumors, including melanoma, seminoma, tetratocarcinoma, neuroblastoma and glioma;
- tumors of the central and peripheral nervous system, including astrocytoma, neuroblastoma, glioma, and schwannomas;
- tumors of mesenchymal origin, including fibrosarcoma, rhabdomyoscaroma, and osteosarcoma; and
- other tumors, including melanoma, xenoderma pigmentosum, keratoactanthoma, seminoma, thyroid follicular cancer and teratocarcinoma.

Compounds of formulas I - IV may also inhibit tumor angiogenesis, thereby affecting the growth of tumors. Such anti-angiogenesis properties of the compounds of formulas I and IV may also be useful in the treatment of certain forms of blindness related to retinal vascularization, arthritis, especially inflammatory arthritis, multiple sclerosis, restinosis and psoriasis.

Compounds of formulas I - IV may induce or inhibit apoptosis, a physiological cell death process critical for normal development and homeostasis. Alterations of apoptotic pathways contribute to the pathogenesis of a variety of human diseases. Compounds of I - IV, as modulators of apoptosis, will be useful in the treatment of a variety of human diseases with aberrations in apoptosis including cancer (particularly, but not limited to follicular lymphomas, carcinomas with p53 mutations, hormone dependent tumors of the breast, prostrate and ovary, and precancerous lesions such as familial adenomatous polyposis), viral infections (including but not limited to herpesvirus, poxvirus, Epstein-Barr virus, Sindbis virus and adenovirus), autoimmune diseases (including but not limited to systemic lupus erythematosus, immune mediated glomerulonephritis, rheumatoid arthritis, psoriasis, inflammatory bowel diseases and autoimmune diabetes mellitus), neurodegenerative disorders (including but not limited to Alzheimer's disease, AIDS-related dementia, Parkinson's disease, amyotrophic lateral sclerosis, retinitis pigmentosa, spinal muscular atrophy and cerebellar degeneration), AIDS, myelodysplastic syndromes, aplastic anemia, ischemic injury associated myocardial infarctions, stroke and reperfusion injury, arrhythmia, atherosclerosis, toxin-induced or alcohol induced liver diseases, hematological diseases (including but not limited to chronic anemia and aplastic anemia), degenerative diseases of the musculoskeletal system (including but not limited to osteoporosis and arthritis), aspirin-sensitive rhinosinusitis, cystic fibrosis, multiple sclerosis, kidney diseases, and cancer pain.

The compounds of this invention are also useful in combination with known anti-cancer and cytotoxic agents and treatments, including radiation. If formulated as a fixed dose, such combination products employ the compounds of this invention within the dosage range described below and the other pharmaceutically active agent within its approved dosage range. Compounds of formulas I - IV can be used sequentially with known anticancer or cytotoxic agents and treatment, including radiation when a combination formulation is inappropriate. Especially useful are cytotoxic drug combinations wherein the second drug chosen acts in a different phase of the cell cycle, e.g. S phase, than the present compounds of formulas I - IV which exert their effects at the G₂-M phase.

The present compounds may exist as multiple optical geometric and stereoisomers. Included within the present invention are all such isomers and mixtures thereof in the racemic form.

The compounds of this invention can be formulated with a pharmaceutical vehicle or diluent for oral, intravenous or subcutaneous administration. The pharmaceutical composition can be formulated in a classical manner using solid or liquid vehicles, diluents and additives appropriate to the desired mode of administration. Orally, the compounds can be administered in the form of tablets, capsules, granules, powders and the like. The compounds are administered in a dosage range of about 0.05 to 200 mg/kg/day, preferably less than 100 mg/kg/day, in a single dose or in 2 to 4 divided doses.

### Method of Preparation

Compounds of formulas II and IV are prepared from compounds of formulas I and III, as shown in Scheme 1. A compound of formula I or III afford compounds of formula II or IV when treated with a reactive metallocene such as titanocene, zirconocene or niobocene (see for example R. Schobert and U. Hohlein, Synlett (1990), 465-466.). Optionally, compounds of formulas II or IV where P₁ and/or P₂ are hydroxyl protecting groups such as silanes, e.g., trialkylsilyl, and the like, can be deprotected by methods known in the art to provide compounds of formula II or IV where where P₁ and P₂ are hydrogen. 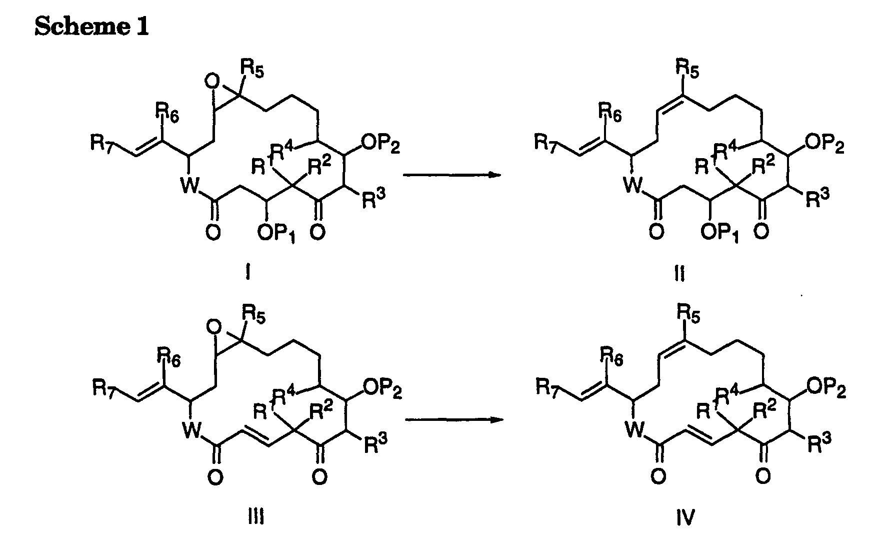 Alternatively, other metal or metal-assisted reagents can be used for the conversion of a compound of formula I or III to a compound of formula II or IV, as listed below:
1) N₂C(CO₂Me)₂, cat Rh₂(OAc)₄
   Martin, M.G.; Ganem, B. *Tett. Lett.* **1984,** *25*, 251.
2) N₂C(CO₂Me)₂, cat [(*n*-C₇H₁₅CO₂)₂Rh]₂
   Raucher, S.; Ki-Whan, C.; Ki-Jun, H.; Burks, J. *J. Org. Chem.* **1986,** *51*, 5503.
3) Zn-Cu, EtOH
   Kupchen, S.M.; Maruyama, M. *J. Org. Chem.* **1971,** *36*, 1187.
4) Mg(Hg), MgBr₂
   Bertini, F.; Grasselli, P.; Zubiani, G.; Cainelli, G. *Chem. Commun*. **1970,** 144.
5) Cr
   Gladysz, J.A.; Fulcher, J.G.; Togashi, S. *J. Org. Chem.* **1976,** *41*, 3647.
6) FeCl₃, *n*-BuLi
   Fujisawa, T.; Sugimoto, K.; Ohta, H. *Chem. Lett.* **1974,** 883.
7) TiCl₃, LiAlH₄
   McMurry, J.E.; Fleming, M.P. *J. Org. Chem*.. **1975,** *40*, 2555.
   McMurry, J.E.; Silvestri, M.G.; Fleming, M.P.; Hoz, T.;
   Grayston, M.W. *J. Org. Chem.* **1978,** *43*, 3249.
8) TiCl₄, Zn
   McMurry, J.E.; Silvestri, M.G.; Fleming, M.P.; Hoz, T.;
   Grayston, M.W. *J. Org. Chem.* **1978,** *43*, 3249.
9) WCl₆, LiAlH₄
   Fugiwara, Y.; Ishikawa, R.; Akiyama, F.; Teranishi, S. *J. Org. Chem.* **1978,** *43*, 2477.
10) NbCl₅, NaAlH₄
   Sato, M.; Oshima, K. *Chem. Lett.* **1982,** 157.
11) VCl₃, Zn
   Inokuchi, T.; Kawafuchi, H.; Torii, S. *Synlett* **1992,** *6*, 510.
12) WCl₆, *n*-BuLi
   Sharpless, K.B.; Umbret, M.A.; Nieh, M.T.; Flood, T.C. *J. Am. Chem. Soc.* **1972,** *94*, 6538.

### Example 1

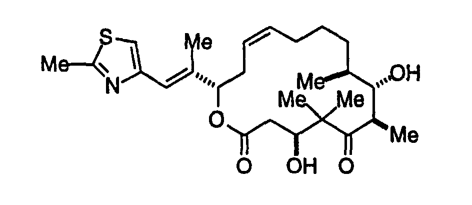

### [4S-[4R,*,7S*,8R*,9R*,15R*(E)]]-4,8-Dihydroxy-5,5,7,9-tetramethyl-16-[1-methyl-2-(2-methyl-4-thiazolyl)ethenyl]-1-oxa-13(Z)-cyclohexadecene-2,6-dione. [Epothilone C]

To a two-necked flask was added chopped pieces of magnesium turnings (24 mg, 1.0 mmol). The flask was flame-dried under vacuum and cooled under argon. Bis(cyclopentadienyl)titanium dichloride (250 mg, 1.0 mmol) was added followed by anhydrous THF (5 mL). The stirring suspension was evacuated with low vacuum, and the reaction flask was refilled with argon. The red suspension became dark, turning a homogeneous deep green after 1.5h with nearly all the magnesium metal being consumed. An aliquot (3.5 mL, 0.70 mmol, 3.5 eq) was removed and cooled to -78 °C under argon. To this solution was added epothilone A (99 mg, 0.20 mmol, 1.0 eq). The reaction mixture was warmed to room temperature and stirred for 15 min. The volatiles were removed *in vacuo* and the residue was chromatographed two times on silica (25g), eluting with 35% EtOAc/hexanes to give 76 mg (80%) of the title compound as a pale yellow viscous oil.

### Example 2

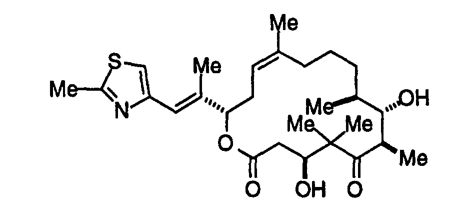

### [4S-[4R*,7S*,8R*,9R*,15R*(E)]]-4,8-Dihydroxy-5,5,7,9,13-pentamethyl-16-[1-methyl-2-(2-methyl-4-thiazolyl)ethenyl]-1-oxa-13(Z)-cyclohexadecene-2,6-dione. [Epothilone D]

To anhydrous THF (5 ml) at -78 °C under argon was added WCl₆ (198 mg, 0.5 mmol) followed by *n*BuLi (0.625 ml of 1.6 *M* solution in hexanes, 1.0 mmol). The reaction was allowed to warm to room temperature over a 20 min period. An aliquot (0.50 ml, 0.05 mmol) of the tungsten reagent was removed and added to epothilone B (9.0 mg, 0.018 mmol) under argon and the reaction mixture was stirred for 15 min, and then quenched by the addition of saturated NaHCO₃ (1 ml). The reaction mixture was extracted with EtOAc (3 x 1 ml), the combined extracts dried (Na₂SO₄), filtered, and the volatiles were removed under vacuum. The residue was chromatographed with 35% EtOAc/hexanes to give the title compound (7.0 mg, 0.014 mmol). MS *m*/*z*: 492.3 (M⁺+H).

### Example 3

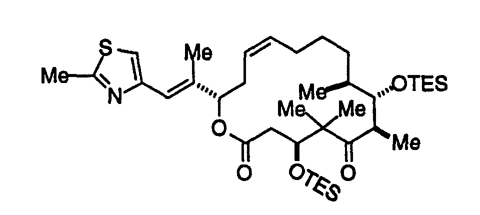

### [4S-[4R*,7S*,8R*,9R*,15R*(E)]]-4,8-Triethylsilyloxy-5,5,7,9-tetramethyl-16-[1-methyl-2-(2-methyl-4-thiazolyl)ethenyl]-1-oxa-13(Z)-cyclohexadecene-2,6-dione. [Bis-Triethylsilyl Epothilone C]

Et₃SiCl (4.15 mmol, 0.700 ml) was added to epothilone A (0.415 mmol, 205 mg), imidazole (2.07 mmol, 140 mg) and *i*-Pr₂EtN (6.22 mmol, 1.08 ml) in DMF (5 ml). The resulting solution was heated at 40 °C. After 16 hrs, additional Et₃SiCl (2.07 mmol, 0.350 ml) and *i*-Pr₂EtN (4.15 mmol, 0.725 ml) were added and the resulting solution stirred at 60 °C for 48 hrs. The reaction was concentrated, and the residue was purified with flash chromatography (10% EtoAc/Hexanes). Bis-triethylsilyl epothilone A was isolated as colorless oil (264 mg, 88%). MS (M⁺+H) 722.

To anhydrous THF (5 ml) at -78 °C under argon was added WCl₆ (198 mg, 0.5 mmol) followed by nBuLi (0.625 ml of 1.6 *M* solution in hexanes, 1.0 mmol). The reaction was allowed to warm to room temperature over a 20 min period. An aliquot (1.0 ml, 0.089 mmol) of the tungsten reagent was removed and added to bis-triethylsilyl epothilone A (22.5 mg, 0.031 mmol) under argon and the reaction stirred for 20 min then quenched by the addition of saturated NaHCO₃ (1 ml). The reaction mixture was extracted with EtOAc (3 x 1 ml), the combined extracts dried (Na₂SO₄), filtered, and the volatiles were removed under vacuum. The residue was chromatographed with 10% EtOAc/hexanes to give the title compound (13.6 mg, 0.019 mmol) in 62% yield. MS *m*/*z*: 706.5 (M⁺+H).

### Example 4

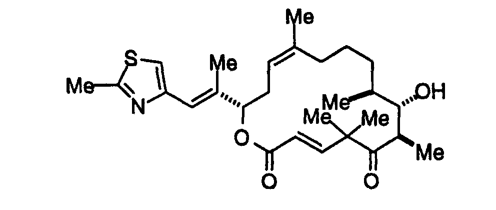

### [7R-[7R*,8S*,9S*,15R*(E)]]-8-Hydroxy-5,5,7,9,13-pentamethyl-16-[1-methyl-2-(2-methyl-4-thiazolyl)ethenyl]-1-oxa-3(E),13(Z)-cyclohexadecadiene-2,6-dione.

The title compound was prepared following the procedure described in Example 2. From 10 mg of [1S-[1R*,3R*(E),10S*,11S*,12R*,16S*]]-11-hydroxy-8,8,10,12,16-pentamethyl-3-[1-methyl-2-(2-methyl-4-thiazolyl)ethenyl]-4,17-dioxabicyclo[14.1.0]heptadec-6-ene-5,9-dione (prepared from epothilone B using the procedure described in WO97/19086 for the analogous conversion of epothilone A), 4.5 mg of title compound was obtained. MS 474 (M+H)⁺.

## Claims

1. A process to produce a compound of the formulas 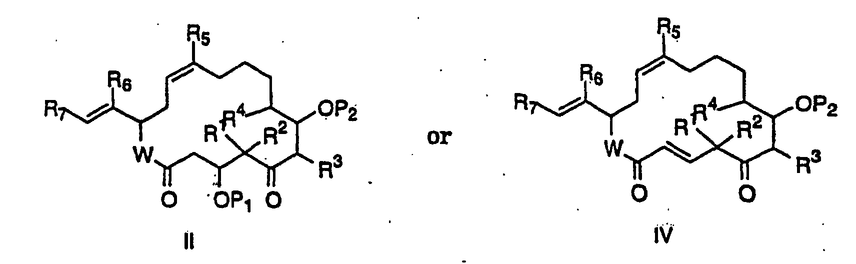 wherein
W is O, or NR₈;
R₁, R₂, R₃, R₄, R₅, R₆, are selected from the group H, alkyl, substituted alkyl, or aryl and when R₁ and R₂ are alkyl can be joined to form a cycloalkyl;
R₇ is selected from the group consisting of H, alkyl, substituted alkyl, aryl, cycloalkyl, or heterocyclo;
R₈ is H, alkyl, or substituted alkyl, OH, O-alkyl, or O-substituted alkyl; and
P₁ and P₂ are selected from the group, H, alkyl, substituted alkyl, alkanoyl, substituted alkanoyl, aroyl, substituted aroyl, trialkylsilyl, aryl dialkylsilyl, diaryl alkylsilyl, or triarylsilyl;
which comprises reacting a compound of the formula 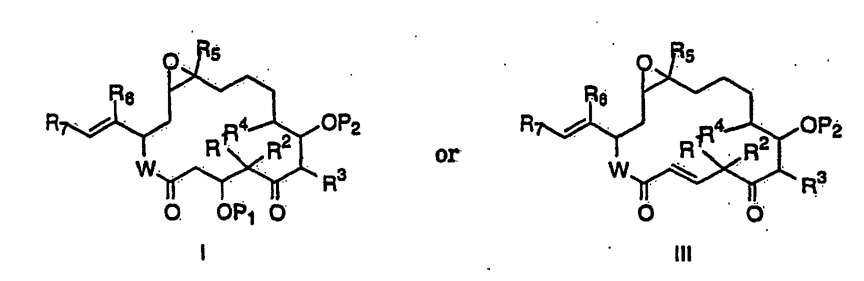 wherein W, R₁, R₂, R₃, R₄, R₅, R₆, R₇, R₈, P₁ and P₂ are as above with a metal or metal assisted reagent selected from the group consisting of a reactive metallocene; [N₂C(CO₂Me)₂, cat Rh₂(OAc)₄]; [N₂C(CO₂Me)₂, cat[(n-C₇H₁₅CO₂)₂Rh]₂]; [Zn-Cu, EtOH]; [Mg(Hg), MgBr]; Cr; [FeCl₃, n-BuLi]; [TiCl₃, LiAlH₄]; [TiCl₄, Zn]; [WCl₆, LiAlH₄]; [NbCl₅, NaAlH₄]; [VCl₃,Zn] and [WCl₆, n-BuLi].

2. The process of claim 1 wherein the metal or metal assisted reagent is a metallocene.

3. The process of claim 2 wherein the metallocene is selected from the group consisting of titanocene, zirconocene or niobocene.

4. The process of claim 1 wherein the metal or metal assisted reagent is [WCl₆, n-BuLi].

## Patentansprüche

1. Verfahren zur Herstellung einer Verbindung der Formel 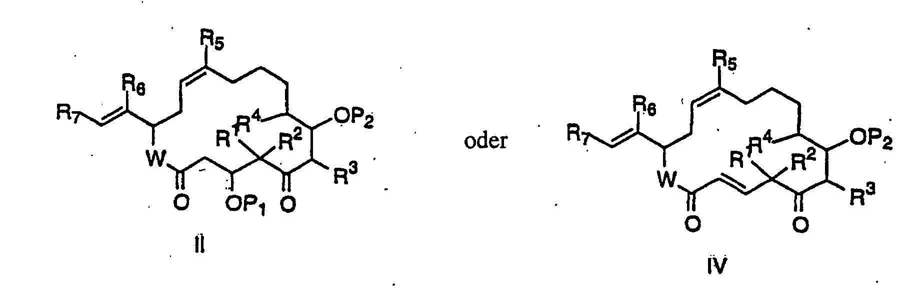 wobei
W die Bedeutung 0 oder NR₈ hat;
R₁, R₂, R₃, R₄, R₅, R₆ aus einem Wasserstoffatom, Alkylrest, substituierten Alkylrest oder Arylrest ausgewählt sind, und falls R₁ und R₂ ein Alkylrest sind, diese zusammengebunden werden können, um einen Cycloalkylrest zu bilden;
R₇ aus einem Wasserstoffatom, Alkylrest, substituierten Alkylrest, Arylrest, Cycloalkylrest oder einem heterocyclischen Rest ausgewählt ist;
R₈ ein Wasserstoffatom, Alkylrest oder substituierter Alkylrest, eine Hydroxygruppe, ein O-Alkylrest oder substituierter O-Alkylrest ist; und
P₁ und P₂ aus einem Wasserstoffatom, Alkylrest, substituierten Alkylrest, Alkanoylrest, substituierten Alkanoylrest, Aroylrest, substituierten Aroylrest, Trialkylsilylrest, Aryldialkylsilylrest, Diarylalkylsilylrest oder Triarylsilylrest ausgewählt sind;
welches das Umsetzen einer Verbindung der Formel 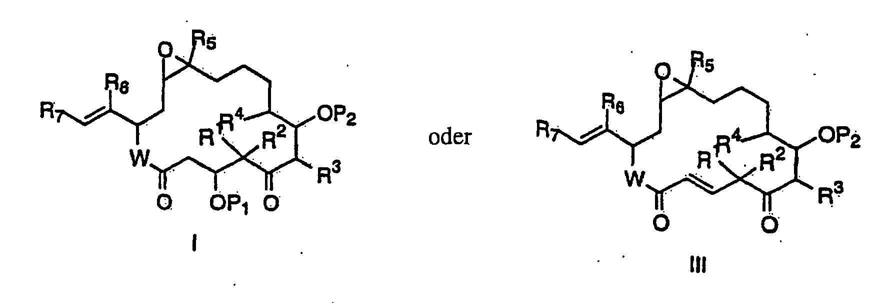 wobei W, R₁, R₂, R₃, R₄, R₅, R₆, R₇, R₈, P₁ und P₂ die vorstehend angegebene Bedeutung haben, mit einem Metall oder einem Metall-unterstützten Reagenz, ausgewählt aus einem reaktiven Metallocen, [N₂C(CO₂Me)₂, kat. Rh₂(OAc)₄]; [N₂C(CO₂Me)₂, kat. [(n-C₇H₁₅CO₂)₂Rh]₂]; [Zn-Cu, EtOH]; [Mg(Hg), MgBr]; Cr; [FeCl₃, n-BuLi]; [TiCl₃, LiAlH₄]; [TiCl₄, Zn]; [WCl₆, LiAlH₄]; [NbCl₅, NaAlH₄]; [VCl₃, Zn] und [WCl₆, n-BuLi], umfasst.

2. Verfahren gemäß Anspruch 1, wobei das Metall oder das Metall-unterstützte Reagenz ein Metallocen ist.

3. Verfahren gemäß Anspruch 2, wobei das Metallocen aus einem Titanocen, Zirconocen oder Niobocen ausgewählt ist.

4. Verfahren gemäß Anspruch 1, wobei das Metall oder das Metall-unterstützte Reagenz [WCl₆, n-BuLi] ist.

## Revendications

1. Procédé pour produire un compose de la formule 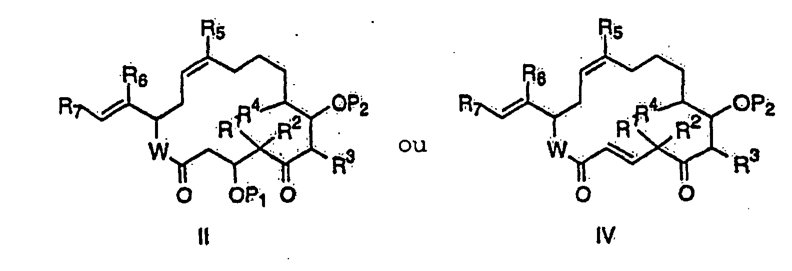 où
W est O, ou NR₈;
R₁,R₂,R₃,R₄,R₅,R₆, sont sélectionnés dans le groupe H, alkyle, alkyle substitué ou aryle et quand R₁ et R₂ sont alkyles, ils peuvent être joints pour former un cycloalkyle.
R₇ est sélectionné dans le groupe consistant en H, alkyle, alkyle substitué, aryle, cycloalkyle ou hétérocyclo ;
R₈ est H, alkyle, ou alkyle substitué, OH, O-alkyle alkyle O-substitué ; et
P₁ et P₂ sont sélectionnés dans le groupe H, alkyle, alkyle substitué, alcanoyle, alcanoyle substitué, aroyle, aroyle substitué, trialkylsilyle, aryl dialkylsilyle, diaryl alkylsilyle, triarylsilyle ;
qui comprend la réaction d'un composé de la formule 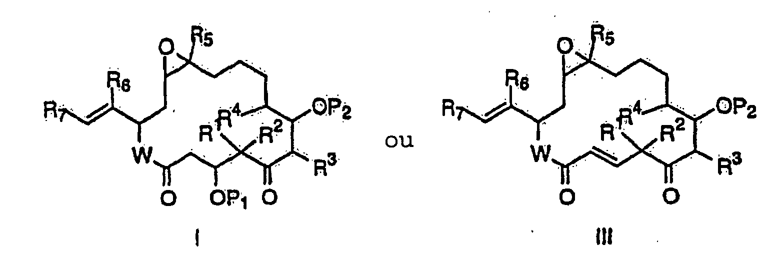 où W, R₁,R₂,R₃,R₄,R₅,R₆,R₇,R₈,P₁ et P₂ sont comme ci-dessus avec un métal ou réactif assisté d'un métal sélectionné dans le groupe consistant en un métallocène réactif; [N₂C(CO₂Me)₂, cat Rh₂(OAc)₄]; [N₂C(CO₂Me)₂, cat[(n-C₇H₁₅CO₂)₂Rh]₂; [Zn-Cu, EtOH]; [Mg(Hg), MgBr]; CR; [FeCl₃,n-BuLi]; [TiCl₃,LiAlH₄]; [TiCl₄,Zn]; [WCl₆,LiAlH₄]; [NbCl₅,NaAlH₄]; [VCl₃,Zn] et [WCl₆,n-BuLi].

2. Procédé de la revendication 1 où le métal ou réactif assisté d'un métal est un métallocène.

3. Procédé de la revendication 2, où le métallocène est sélectionné dans le groupe consistant en titanocène, zirconocène ou niobocène.

4. Procédé de la revendication 1, où le métal ou réactif assisté d'un métal est [WCl₆,n-BuLi].
